# FASCICULE DE BREVET EUROPEEN

(11) **EP 2 160 132 B1**
(45) Date de publication et mention de la délivrance du brevet: **22.08.2012**
(21) Numéro de dépôt: 08806073.6
(22) Date de dépôt: 25.06.2008
(51) Int. Cl.: A61B 5/0404

(54) **ELECTROCARDIOGRAPHE AMBULATOIRE EVENEMENTIEL**
EREIGNISGESTEUERTER AMBULANTER ELEKTROKARDIOGRAPH
EVENT DRIVEN AMBULATORY ELECTROCARDIOGRAPH

(30) Priorité: 26.06.2007 FR 0756044; 26.06.2007 FR 0756045; 26.06.2007 FR 0756048
(43) Date de publication de la demande: 10.03.2010
(73) Titulaire: Parsys, 93885 Noisy Le Grand Cedex (FR)
(72) Inventeur: DANTENY, Alain, F-13700 Marignane (FR)
(74) Mandataire: Delumeau, François Guy
(86) Numéro de dépôt international: PCT/FR2008/051141
(87) Numéro de publication internationale: WO 2009/007592

(56) Documents cités:
- EP-A- 1 676 528
- EP-B- 0 611 287
- WO-A-99/38436
- WO-A-2005/027720
- US-A- 4 573 474
- US-A- 5 010 888

## Description

### Arrière-plan de l'invention

La présente invention se rapporte au domaine général de l'électrocardiographie.

L'invention concerne plus particulièrement un électrocardiographe ambulatoire événementiel. Un tel dispositif permet avantageusement à un patient d'enregistrer lui-même, par exemple à son domicile, un électrocardiogramme d'urgence en cas de crise supposée sans la présence ni l'assistance d'un personnel médical qualifié. Il doit être d'encombrement réduit et simple de maniement afin d'être positionné facilement sur le thorax du patient dans une position suffisamment stable pour permettre une bonne acquisition des signaux sans engendrer de stress supplémentaire pour le patient.

Le document EP 0 611 287 B1 décrit un électrocardiographe portatif permettant la saisie d'un électrocardiogramme à douze dérivations à partir de signaux acquis par neuf électrodes. Un électrocardiogramme à douze dérivations permet de diagnostiquer différentes pathologies cardiaques ou cardio-vasculaires telles des arythmies ou des anomalies de repolarisation.

Les signaux représentatifs de l'activité cardiaque du patient peuvent être mémorisés dans l'électrocardiographe pour reproduire l'électrocardiogramme sur une bande d'enregistrement. Ils peuvent aussi être convertis en signaux acoustiques et transmis par un haut-parleur par une ligne téléphonique à un centre distant.

Dans le mode de réalisation représenté sur la **figure 1A****,** l'électrocardiographe portatif 100 du document EP 0 611 287 B1 est équipé d'un boîtier comportant une surface support rigide 114 sur laquelle sont disposées six électrodes précordiales 101-106 destinées à être appliquées respectivement sur les points V1-V6 du thorax du patient.

La surface support 114 est concave. Au sens de l'invention, une surface « concave » ou « sensiblement concave » désigne une surface apte à épouser le thorax bombé d'un patient et non nécessairement une surface concave au sens mathématique strict. En l'espèce, la surface concave 114 de l'électrocardiographe 100 est composée de trois sections, à savoir un support principal 111 et deux supports latéraux 112 et 113, chaque support latéral formant un angle fixe α, β avec le support principal 111.

Il n'est pas simple, pour un patient utilisant un tel dispositif de bien positionner les électrodes précordiales sur son thorax, notamment au moment d'une crise. En particulier, l'électrocardiographe 100 ne permet pas de garantir une mise en contact « parfaite » des électrodes précordiales avec les points précordiaux du thorax du patient de sorte à pouvoir saisir correctement l'électrocardiogramme. Or, la qualité de l'électrocardiogramme enregistré dépend en grande partie des conditions d'application des électrodes sur le corps du patient, et notamment des électrodes précordiales plus sensibles à un mauvais positionnement. En cas de mauvais positionnement et notamment de mauvaise mise en contact des électrodes avec les points précordiaux correspondants, les signaux électriques acquis par les électrodes peuvent être fortement perturbés et ne pas permettre une exploitation satisfaisante de l'électrocardiogramme ainsi saisi.

Ceci est encore plus préjudiciable lorsque ces signaux de mauvaise qualité sont transmis sur une ligne téléphonique pour l'établissement de l'électrocardiogramme par un centre distant, le patient n'ayant pas la possibilité de se rendre compte immédiatement que l'électrocardiogramme est inexploitable. Au surplus, le coût de la communication téléphonique est une pure perte.

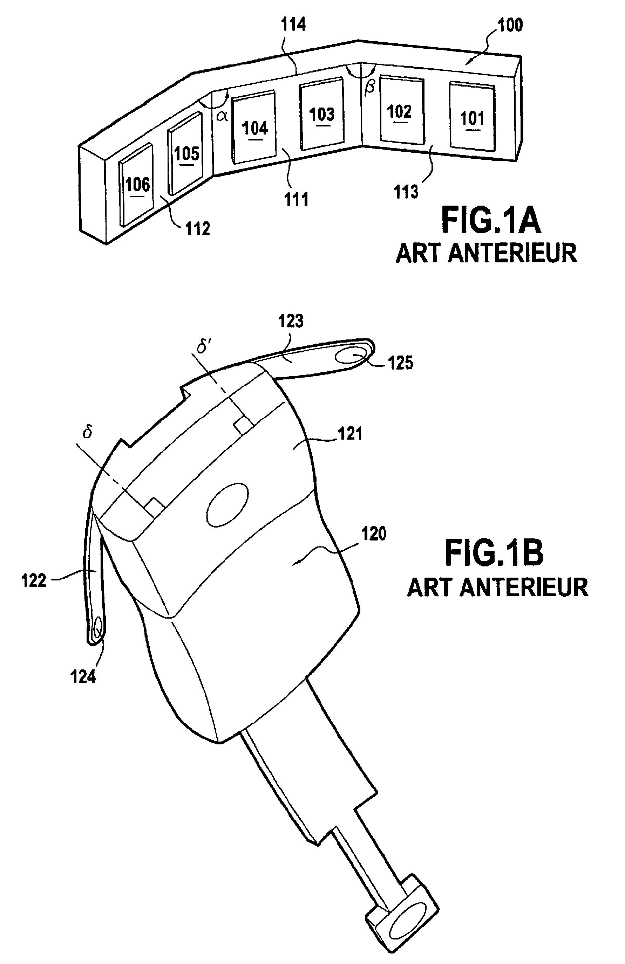

Afin de minimiser cette difficulté, le document WO 99/38436 propose un électrocardiographe ambulatoire à trois électrodes précordiales, représenté dans un mode de réalisation sur la **figure 1****B.** Ce dispositif 120 permet l'enregistrement d'un électrocardiogramme à huit dérivations au lieu de douze classiquement.

L'électrocardiographe 120 est dans ce mode de réalisation équipé de deux bras 122 et 123 munis chacun d'une électrode précordiale (124 et 125 respectivement) à leur extrémité. La troisième électrode précordiale est située sur la face inférieure 121.

Les bras 122 et 123 peuvent pivoter respectivement autour de rotules définies par les axes δ et δ' perpendiculaires à la face inférieure 121 pour se rabattre sur la face supérieure de l'électrocardiographe 120. Ceci permet de réduire l'encombrement de l'électrocardiographe 120 lorsque celui-ci n'est pas en usage.

Cependant, l'analyse d'un électrocardiogramme à huit dérivations tel que celui décrit dans le document WO 99/38436 ne peut être réalisée par une personne non formée à l'interprétation de ces résultats, tel un médecin urgentiste ou généraliste, même lorsque celui-ci est habitué à Interpréter des électrocardiogrammes à douze dérivations. Par ailleurs, la réglementation en vigueur actuelle en France (notamment pour la Sécurité Sociale) ne reconnaît pas l'utilisation de tels électrocardiogrammes.

### Objet et résumé de l'invention

La présente invention a pour but principal de palier les inconvénients précités.

Plus précisément, l'invention concerne un électrocardiographe ambulatoire événementiel adapté à saisir un électrocardiogramme à douze dérivations à partir de signaux électriques analogiques représentatifs de l'activité cardiaque d'un patient acquis à l'aide d'au moins neuf électrodes, cet électrocardiographe comportant un support principal et au moins un support latéral sur lesquels sont réparties six électrodes précordiales, chaque support latéral formant un angle avec le support principal de sorte que le support principal et les supports latéraux définissent une surface sensiblement concave. Conformément à l'invention, le support principal de l'électrocardiographe selon l'invention est une face frontale d'un corps de l'électrocardiographe et chaque support latéral est un bras pouvant être replié le long du corps et rotatif autour d'un axe sensiblement parallèle à la face frontale, chaque bras étant adapté à former un angle d'ouverture variable avec la face frontale.

La surface sensiblement concave peut ainsi s'adapter à la morphologie du patient et permettre le bon positionnement des électrodes précordiales sur le thorax du patient.

Conformément à l'invention, l'électrocardiographe selon l'invention comporte en outre des moyens permettant de maintenir le bon positionnement des électrodes précordiales sur le thorax du patient.

Ainsi, grâce à l'utilisation de bras rotatifs munis d'électrodes précordiales (sans câble) et pouvant former un angle d'ouverture variable avec la face frontale du corps de l'électrocardiographe, l'électrocardiographe selon l'invention permet de saisir un électrocardiogramme pour des patients présentant des morphologies différentes. Cette saisie est réalisée par simple application de l'électrocardiographe sur le thorax des patients. Le bon positionnement des électrodes précordiales sur le thorax du patient, et notamment la qualité du contact de ces électrodes avec les points correspondants du thorax, est assuré d'une part par l'usage de tels bras et d'autre part par des moyens permettant de maintenir ce bon positionnement et ce contact. Ceci permet de saisir un électrocardiogramme de bonne qualité.

Chaque bras de l'électrocardiographe selon l'invention est équipé d'un ressort. Ce ressort assure une certaine « élasticité » du bras autour d'un angle d'ouverture adapté à la morphologie du patient. Cette élasticité permet de garder les électrodes précordiales en contact avec les points précordiaux du thorax du patient et d'assurer la stabilité des signaux saisis une fois les électrodes correctement positionnées. Notamment, cela permet de ne pas exercer de pression supplémentaire sur les électrodes précordiales pour garantir leur contact avec le thorax du patient, une telle pression pouvant perturber la saisie de l'électrocardiogramme.

Par ailleurs, chaque bras pouvant être replié le long du corps, l'encombrement de l'électrocardiographe selon l'invention est réduit.

Dans un mode particulier de réalisation, les douze dérivations de l'électrocardiogramme sont acquises simultanément, c'est-à-dire que l'électrocardiographe selon l'invention est un électrocardiographe « 12 dérivations - 12 pistes ». Ceci permet de délivrer un électrocardiogramme précis, utile pour le diagnostic d'anomalies cardiaques, comportant douze dérivations capturant l'activité cardiaque du patient au même instant.

Les électrodes de l'électrocardiographe sont préférentiellement des électrodes sèches. De telles électrodes, contrairement aux électrodes gélifiées ou collantes, ne requièrent aucune préparation de la peau ni d'application de gel (ex. gel électrolytique). Ceci permet d'éviter les effets secondaires habituellement observés avec les électrodes gélifiées ou collantes, tels que les problèmes d'irritation ou d'allergie.

l'électrocardiographe comporte deux bras de part et d'autre du corps, chaque bras étant muni de deux électrodes précordiales.

La disposition des électrodes précordiales sur les deux bras et sur la face frontale de l'électrocardiographe est par exemple la suivante :
- les deux électrodes précordiales de l'un des bras correspondent respectivement aux points V1 et V2 du thorax du patient ;
- les deux électrodes précordiales de l'autre bras correspondent respectivement aux points V5 et V6 du thorax du patient ; et
- la face frontale du corps de l'électrocardiographe est munie de deux électrodes précordiales correspondant respectivement aux points V3 et V4 du thorax du patient.

L'électrocardiographe permet ainsi d'acquérir directement les dérivations précordiales V1 à V6 connues de l'homme du métier par simple application de l'électrocardiographe sur le thorax du patient.

Dans un mode particulier de réalisation, deux électrodes parmi les neuf électrodes de l'électrocardiographe selon l'invention permettent l'acquisition de signaux relatifs aux membres supérieurs du patient.

Préférentiellement, ces deux électrodes sont disposées sur l'électrocardiographe de façon à permettre l'application des index ou des majeurs du patient sur ces deux électrodes lorsque les six électrodes précordiales sont positionnées sur le thorax du patient.

Ceci permet avantageusement au patient une bonne préhension et un bon maintien de l'électrocardiographe pour la saisie de l'électrocardiogramme tout en gardant une position détendue. En particulier, cela permet d'éviter que le patient ait les muscles des épaules tendus, ce qui pourrait créer des signaux parasites au cours de la saisie de l'électrocardiogramme.

Dans un mode particulier de réalisation, l'une des neuf électrodes de l'électrocardiographe selon l'invention est une électrode de type cuillère ou palette se trouvant à l'extrémité d'un cordon attaché à l'électrocardiographe et permettant l'acquisition du signal relatif au membre inférieur gauche du patient.

Une telle électrode garantit un meilleur contact avec le corps du patient qu'une électrode quille par exemple, qui peut aisément rouler. Il est facile notamment de maintenir plaquée une telle électrode sur le corps du patient à l'aide de ses sous-vêtements, afin d'acquérir le signal relatif au membre inférieur gauche de ce patient.

Dans un mode particulier de réalisation, l'électrocardiographe comporte en outre en sus des neuf électrodes une électrode neutre positionnée sur la face frontale du corps de l'électrocardiographe de sorte à être appliquée sous le mamelon gauche du patient.

Cette électrode remplace l'électrode du membre inférieur droit classiquement utilisée comme neutre dans les électrocardiographes de l'art antérieur. Comme les électrodes précordiales, l'électrode neutre est sans câble et préférentiellement sèche.

Elle peut également par ailleurs du fait de sa position, constituer un repère pour le patient pour positionner l'électrocardiographe selon l'invention sur son thorax. En variante, l'électrocardiographe peut comporter un repère visuel ou tactile permettant au patient de le positionner correctement sur son thorax.

Dans un mode de réalisation particulier, l'électrocardiographe ambulatoire événementiel comporte, pour chacune de ses électrodes :
- des moyens pour convertir le signal analogique acquis par cette électrode en signal numérique ;
- des moyens pour mémoriser le signal numérique ;
- des moyens pour convertir le signal numérique en signal analogique et pour rediriger ce signal analogique vers une fiche compatible avec un premier connecteur d'un brin dont un deuxième connecteur est compatible avec un deuxième électrocardiographe.

Les signaux analogiques d'un électrocardiographe peuvent ainsi être fournis à un électrocardiographe traditionnel apte à restituer les douze dérivations précitées.

Dans ce mode d'utilisation, le patient qui a saisi son électrocardiographe lui-même en ambulatoire peut se rendre chez un médecin avec son électrocardiographe, le médecin connectant cet électrocardiographe avec son propre électrocardiographe pour obtenir l'électrocardiogramme.

Dans un mode particulier de réalisation, l'électrocardiographe événementiel ambulatoire comporte :
- des moyens aptes à déterminer si les signaux analogiques comportent des caractéristiques correspondant à au moins un complexe QRS, de tels signaux étant dits "conformes" ; et le cas échéant
- une mémoire apte à mémoriser ces signaux conformes.

Ainsi et de façon très avantageuse, cet électrocardiographe ne mémorise pas les signaux obtenus par les électrodes tant que ceux-ci ne sont pas conformes, notamment lorsqu'ils ne sont pas encore stabilisés pendant la phase de positionnement ou de calage de l'électrocardiographe sur le thorax du patient, ou en cas de mouvement important ou brusque du patient pendant l'acquisition.

Les caractéristiques d'un complexe QRS peuvent par exemple être des caractéristiques de durée ou d'amplitude. L'homme du métier connaît ces caractéristiques et elles ne seront pas rappelées ici.

On garantit ainsi que les signaux mémorisés seront d'une qualité suffisante pour permettre la restitution d'un électrocardiogramme exploitable.

Dans un mode de réalisation, l'électrocardiographe comporte des moyens de filtrage de ces signaux conformes.

Ces moyens de filtrage sont par exemple au moins adaptés à éliminer des signaux parasites ou à stabiliser la ligne de base. Ils peuvent en particulier mettre en oeuvre l'algorithme connu de Pan Tompkins.

Dans un mode particulier de réalisation, l'électrocardiographe événementiel ambulatoire comporte en outre :
- des moyens de télécommunication aptes à transmettre les signaux conformes ou filtrés à un centre distant à partir de cette mémoire ; et
- des moyens de contrôle aptes à déclencher cette transmission sur détection d'au moins un événement prédéterminé.

De façon très avantageuse, cet électrocardiographe ne transmet au centre distant que des signaux exploitables.

Dans un mode particulier de réalisation, les moyens de contrôle sont adaptés à déclencher la transmission dès que lesdits signaux ont été déterminés comme étant conformes ou dès qu'ils ont été filtrés.

Autrement dit, si les signaux acquis par les électrodes ne sont pas exploitables, ils ne sont pas transmis au centre distant.

Dans un mode particulier de réalisation, les moyens de contrôle sont adaptés à déclencher la transmission suite à la réception, par l'électrocardiographe selon l'invention, d'une commande à cet effet.

Une telle commande peut notamment être émise par le centre distant lorsque celui-ci a détecté une erreur dans des signaux précédemment reçus, par exemple en raison d'une erreur de transmission.

Préférentiellement les signaux sont mémorisés dans l'électrocardiographe en attente d'une éventuelle commande. Dans un mode particulier de réalisation de l'invention, la mémoire comporte des signaux correspondant à plusieurs acquisitions. Préférentiellement, lorsque la mémoire est saturée, les signaux de la dernière acquisition viennent écraser ceux de l'acquisition la plus ancienne.

Dans un mode particulier de réalisation, les moyens de contrôle sont aptes à empêcher la transmission précitée.

Ce mode permet de forcer la conservation des signaux en mémoire. Il peut notamment être utilisé lorsque le patient à l'intention de se rendre chez son médecin pour lui fournir les signaux correspondant à une acquisition particulière.

Dans un mode particulier de réalisation, l'électrocardiographe comporte des moyens secondaires d'obtention d'une pluralité de signaux représentatifs de l'activité cardiaque d'un patient en provenance d'un autre dispositif.

Ce mode de réalisation permet notamment de mémoriser ou de transmettre des signaux acquis avec un autre dispositif, par exemple une ceinture à électrodes.

Ce mode de réalisation permet ainsi de mettre en place un monitoring du patient à distance.

Dans un mode particulier de réalisation, ces moyens secondaires comportent une pluralité de fiches, chacune étant compatible avec un connecteur d'un brin apte à véhiculer un signal analogique obtenu par ce dispositif.

Le patient peut ainsi en particulier effectuer une acquisition avec une ceinture à électrodes, après avoir pris soin de connecter les connecteurs des brins de la ceinture dans les fiches de l'électrocardiographe pour transmettre les signaux acquis par cette ceinture au centre distant.

Dans un mode particulier de réalisation, l'électrocardiographe ambulatoire événementiel comporte en outre des moyens pour afficher un message représentatif du bon et/ou du mauvais positionnement des électrodes.

Cet affichage peut rassurer le patient et ainsi faire baisser son niveau de stress, au bénéfice de la qualité des signaux acquis.

Il est également possible de mettre en oeuvre un système de surveillance à distance de l'activité cardiaque d'un patient comportant :
- un électrocardiographe tel que mentionné ci dessus, dans lequel les moyens de télécommunication sont constitués par des moyens de télécommunication sans fil avec une base connectée à un réseau de télécommunication; et
- cette base.

On obtient ainsi un électrocardiographe d'utilisation très souple puisque le patient n'est pas obligé de se trouver à côté d'une prise réseau typiquement une prise téléphonique pour effectuer son acquisition.

Les moyens de télécommunication sans fil sont par exemple conformes à la norme Bluetooth (marque déposée).

Dans un mode particulier de réalisation, le système de surveillance comporte en outre un équipement portatif apte à établir une communication vocale, via cette base, avec un opérateur distant. Cet équipement peut notamment être de la forme d'un médaillon.

L'opérateur distant peut ainsi guider le patient dans l'utilisation de son électrocardiographe et le rassurer en cas de stress. Cette communication peut également être une communication Bluetooth (marque déposée).

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- la figure 1A, déjà décrite, représente une vue schématique d'un dispositif de l'art antérieur décrit dans le document EP 0 611 287 B1 dans un mode particulier de réalisation ;
- la figure 1B, déjà décrite, représente une vue schématique d'un dispositif de l'art antérieur décrit dans le document WO 99/38436 dans un mode particulier de réalisation ;
- la figure 2 représente une première vue en perspective de l'électrocardiographe, lorsque ses bras sont repliés de part et d'autre du corps, dans un mode particulier de réalisation ;
- la figure 3 représente une seconde vue en perspective de l'électrocardiographe, lorsque ses bras sont repliés de part et d'autre du corps, dans un mode particulier de réalisation ;
- la figure 4 représente une troisième vue en perspective de l'électrocardiographe, notamment lorsque ses bras sont dépliés, dans un mode particulier de réalisation ;
- la figure 5 représente le positionnement des points précordiaux V1 à V6 sur le thorax d'un patient ;
- la figure 6 représente une vue de dessus de l'électrocardiographe dans un mode particulier de réalisation, sur laquelle plusieurs positions des bras de l'électrocardiographe sont représentées ;
- la figure 7 représente une vue schématique du positionnement de l'électrocardiographe lors de son utilisation pour la saisie d'un électrocardiogramme, dans un mode particulier de réalisation ;
- la figure 8A représente un électrocardiographe conforme à l'invention, dans un mode particulier de réalisation ;
- la figure 8B représente en détails des liaisons électriques de l'électrocardiographe de la figure 8A ; et
- la figure 9 représente un système de surveillance à distance de l'activité cardiaque d'un patient

### Description détaillée de plusieurs modes de réalisation

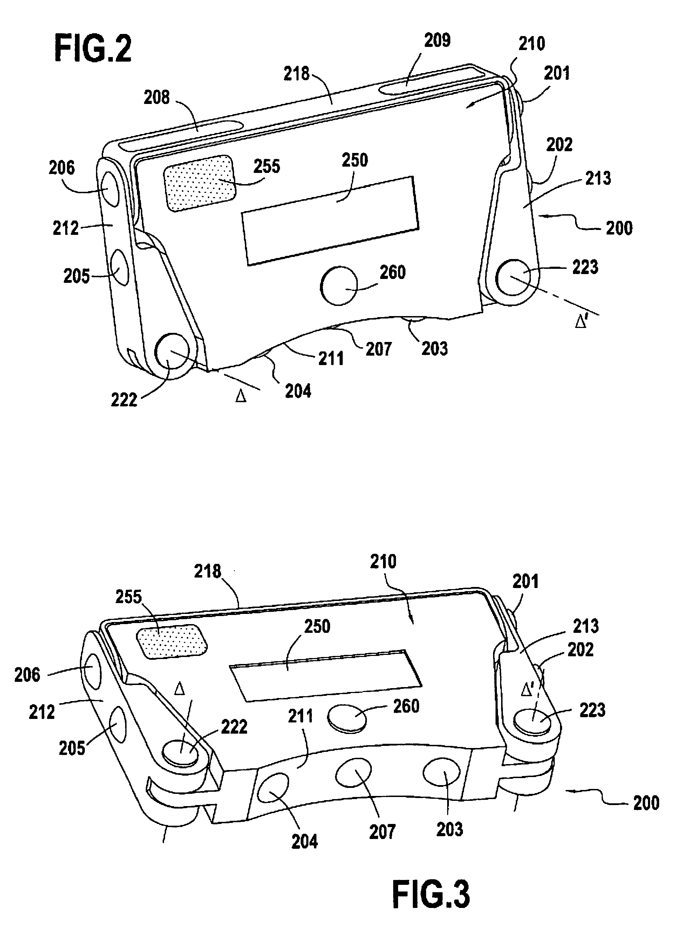

Les **figures 2, 3** **et** **4** représentent un électrocardiographe ambulatoire événementiel 200 dans un mode particulier de réalisation. Un tel électrocardiographe permet à un patient d'enregistrer lui-même, par exemple à son domicile, un électrocardiogramme d'urgence, en cas de crise supposée, sans nécessiter l'assistance d'un personnel médical qualifié.

L'électrocardiographe 200 comporte un corps 210 comprenant notamment une face frontale 211, une face supérieure 215, une face inférieure 216 et une face arrière 217. Dans l'exemple décrit ici, la face frontale 211 est elle-même une surface sensiblement concave sur laquelle sont disposées trois électrodes 203, 204 et 207. Elle constitue un support principal au sens de l'invention. En variante, la face frontale avant 211 peut être plane.

Dans l'exemple décrit ici, l'électrocardiographe 200 comporte également deux bras 212 et 213 (supports latéraux au sens de l'invention) pouvant être repliés le long du corps 210. Ces bras 212 et 213 sont articulés : chaque bras est en effet adapté à effectuer une rotation autour d'un axe de rotation sensiblement parallèle à la face frontale 211, de sorte à former un angle d'ouverture variable avec la face frontale 211. Ainsi, le bras 212 est rotatif autour de l'axe Δ, tandis que le bras 213 est rotatif autour de l'axe Δ'.

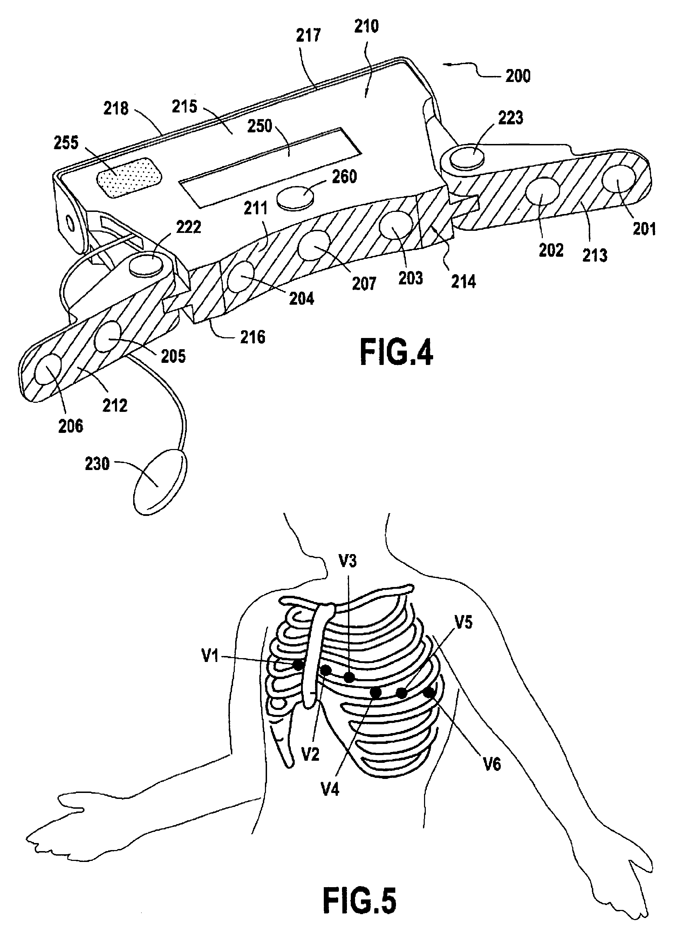

Cette rotation autour des axes Δ et Δ' est permise dans l'exemple décrit ici par les goupilles 222 et 223 respectivement. A titre d'exemple, les figures 2 et 3 représentent les bras 212 et 213 en position repliée, par exemple lorsque l'électrocardiographe 200 n'est pas en fonctionnement ou en mode de saisie d'un électrocardiogramme. La figure 4 quant à elle, illustre les bras 212 et 213 en position dépliée, lorsque l'électrocardiographe 200 est prêt à être utilisé pour la saisie d'un électrocardiogramme. Dans une telle position, le bras 212, la face frontale 211 et le bras 213 forment une surface 214 sensiblement concave, représentée de manière hachurée sur la figure 4.

Dans l'exemple décrit ici, deux électrodes 201 et 202, respectivement 205 et 206, sont disposées sur le bras 213, respectivement sur le bras 212. Comme les électrodes 203, 204 et 207 disposées sur la face frontale 211, les électrodes 201, 202, 205 et 206 sont ici des électrodes sèches. De telles électrodes sont connues de l'homme du métier et ne seront pas décrites plus en détails ici. Chaque électrode est en laiton chromé et présente, dans l'exemple décrit ici, un diamètre d'environ 20 mm. Ces électrodes sont sans câble.

Les électrodes 201, 202, 203, 204, 205 et 206 sont des électrodes précordiales destinées à être disposées sur les points V1 à V6 respectivement de l'aire précordiale du thorax du patient. La disposition de ces points, connue de l'homme du métier, est représentée sur la **figure 5****.**

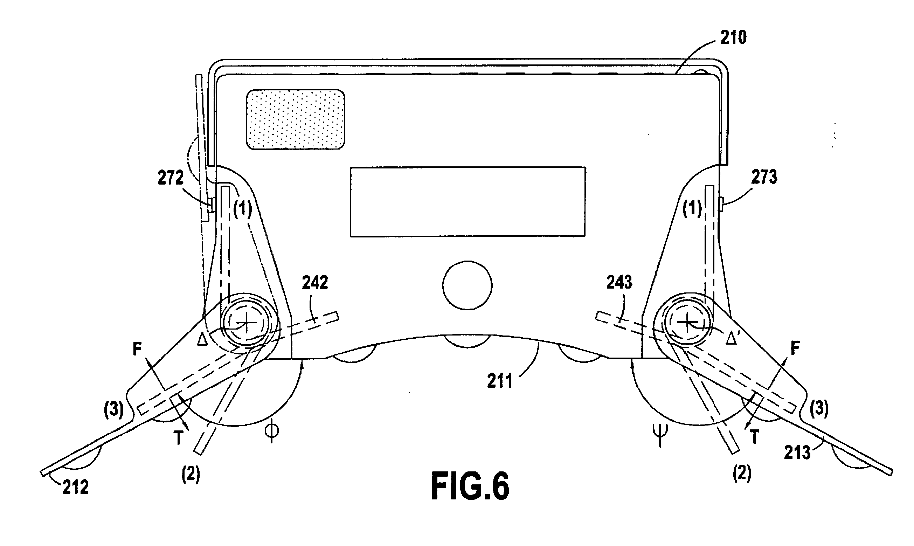

Ainsi, la disposition des électrodes sur les bras 212 et 213 et sur la face latérale 211, ou de façon équivalente sur la surface sensiblement concave 214 est telle que les électrodes 201, 202, 203, 204, 205 et 206 puissent être mises en contact avec les points V1 à V6 du patient pour la saisie de l'électrocardiogramme. Les bras articulés 212 et 213 forment des angles d'ouverture variables de part et d'autre de la face frontale 211 (angles φ et ψ représentés sur la figure 6) de sorte à pouvoir s'adapter à la morphologie du patient afin que les électrodes soient correctement positionnées sur les points précordiaux V1 à V6 de ce patient.

L'électrode 207 placée sur la face frontale 211 à mi-distance entre les électrodes précordiales 204 et 203 constitue une électrode dite neutre, utilisée pour le calcul des douze dérivations de l'électrocardiogramme (D₁, D2, D3, aVR, aVL, aVF, V1, V2, V3, V4, V5, V6) selon des principes connus de l'homme du métier. Elle se situe ici au centre de la face frontale 211.

Afin de maintenir le bon positionnement des électrodes sur les points précordiaux V1 à V6 et en particulier un contact « parfait » entre les électrodes et le thorax du patient en ces points précis, l'électrocardiographe 200 comporte par ailleurs des moyens pour maintenir ce bon positionnement. Ceci permet d'assurer une bonne saisie de l'électrocardiogramme en minimisant ou en supprimant les signaux parasites liés à un mauvais contact entre les électrodes et le thorax du patient. Dans l'exemple décrit ici, ces moyens comportent deux ressorts, ou plus précisément un ressort associé à chaque bras, comme représenté sur la **figure 6** décrite maintenant.

Les ressorts 242 et 243 sont associés respectivement aux bras 212 et 213. Chaque ressort est fixé par une extrémité « fixe » au corps 210 de l'électrocardiographe 200 et par l'autre extrémité au bras auquel il est associé. Cette extrémité est « mobile » du fait de la mobilité des bras 212 et 213 autour de leur axe de rotation respectif.

En référence à la figure 6, lorsque les ressorts 242 et 243 sont au repos, les bras sont en position (2).

Lorsque l'électrocardiographe 200 n'est pas en fonctionnement ou en mode de saisie d'un électrocardiogramme, les bras 212 et 213 sont ramenés le long du corps 210 en position (1). Cette position est maintenue par exemple ici à l'aide de deux aimants 272 et 273 localisés de part et d'autre du corps 210 et qui permettent d'exercer une force inverse à la tension T du ressort. Lors de la mise en marche de l'électrocardiographe 200, par exemple par une pression exercée par le patient sur un bouton de mise en marche, l'action des aimants 272 et 273 est annulée ce qui permet de relâcher les bras 212 et 213 qui prennent alors leurs positions de repos (2).

Lorsqu'on applique l'électrocardiographe 200 sur le thorax d'un patient, une force F est exercée par le thorax du patient à l'encontre de la tension T du ressort 242, respectivement du ressort 243, de sorte que le bras associé au ressort 242, respectivement au ressort 243, soit maintenu hors de sa position de repos (2) dans une position contrainte (3). Le bras 212, respectivement 213, forme alors un angle d'ouverture φ, respectivement ψ, avec la face frontale 211. Ceci permet de garantir un bon contact entre les électrodes situées sur les bras et le thorax du patient quelle que soit la morphologie du patient. La position de repos de chaque ressort est fixée ici en amont lors de la fabrication de l'électrocardiographe 200 de façon à permettre ce fonctionnement quelle que soit la morphologie du patient.

Dans un autre mode de réalisation, afin de maintenir le bon positionnement des électrodes sur le thorax du patient, on peut également utiliser un dispositif cranté associé à chacun des bras (ex. une roue crantée sur chaque bras).

L'électrocardiographe 200 comporte par ailleurs un capot 218 fixé au corps 210 et articulé par rapport à ce corps 210 de façon à pouvoir basculer en direction de la face inférieure 216. La face inférieure 216 de l'électrocardiographe 200 est par définition ici la face opposée à la face supérieure 215 (cf. figure 4).

Sur le capot 218, sont disposées, à chaque extrémité (cf. figure 2), deux électrodes périphériques 208 et 209 destinées à acquérir les signaux relatifs aux membres supérieurs du patient (membre supérieur gauche VL et membre supérieur droit VR). Dans l'exemple décrit ici, ces électrodes 208 et 209 sont des électrodes sèches métalliques en laiton chromé et sans câble. Elles présentent une forme oblongue pour que le patient puisse y appliquer ses index ou ses majeurs. Ceci lui permet avantageusement de pouvoir tenir le dispositif 200 de manière stable, les muscles des épaules détendus, pour une bonne saisie de l'électrocardiogramme une fois les six électrodes précordiales correctement positionnées sur les points précordiaux V1 à V6.

L'électrocardiographe 200 comporte également une troisième électrode périphérique 230 sèche, préférentiellement de type cuillère ou palette, se trouvant à l'extrémité d'un cordon métallique attaché à l'électrocardiographe 200 (cf. figure 4). Cette électrode est appliquée dans la région de l'aine pour l'acquisition du signal relatif au membre inférieur gauche (VF). Dans l'exemple décrit ici, le cordon métallique au bout duquel se trouve l'électrode 230 peut être enroulé lorsque l'électrocardiographe 200 n'est pas en fonctionnement de sorte à être rangé à l'intérieur du corps 210, par exemple dans une fente se trouvant sur une face latérale du corps 210 de l'électrocardiographe 200 (sous le bras 212 lorsque celui-ci est en position repliée).

Dans un autre mode de réalisation, une électrode sèche de type quille peut également être utilisée comme électrode périphérique afin d'acquérir le signal relatif au membre inférieur gauche (VF).

Les six électrodes précordiales 201 à 206, les trois électrodes périphériques 208, 209 et 230 et l'électrode « neutre » 207 sont utilisées pour l'acquisition de signaux représentatifs de l'activité cardiaque du patient permettant à l'électrocardiographe ambulatoire événementiel 200 de délivrer un électrocardiogramme à douze dérivations. Dans l'exemple décrit ici, on suppose par ailleurs que les douze dérivations sont acquises (via les dix électrodes) et délivrées simultanément. Ceci permet de délivrer un électrocardiogramme très précis, pour lequel les différentes dérivations représentent une capture de l'activité cardiaque du patient à un même instant. On parle alors d'électrocardiographe « 12 dérivations - 12 pistes ».

L'électrocardiographe 200 comporte également un écran graphique 250 permettant d'afficher des messages notamment pour signaler un mauvais positionnement des électrodes. Par exemple, l'électrocardiographe 200 comporte des moyens pour analyser le signal reçu via les électrodes et détecter si l'une des électrodes n'est pas en contact avec le corps du patient. Le cas échéant, un message signalant ce mauvais positionnement est affiché sur l'écran 250. D'autres types de message peuvent être affichés sur cet écran, comme par exemple des messages liés à l'utilisation de l'appareil.

Ces messages sont par ailleurs diffusés à l'aide d'un haut-parleur 255 disposé sur la face supérieure 215 du corps 210.

L'électrocardiographe 200 dispose également d'un bouton 260 de marche/arrêt visant à mettre en marche l'électrocardiographe 200 pour la saisie d'un électrocardiogramme ou à l'éteindre. Comme vu précédemment, lorsque le patient appuie sur ce bouton 260, les aimants 272 et 273 sont désactivés de sorte que les bras 212 et 213 prennent une position de repos, prêts à être positionnés sur le thorax du patient pour que les six électrodes précordiales 201-206 soient mises en contact avec les points V1-V6.

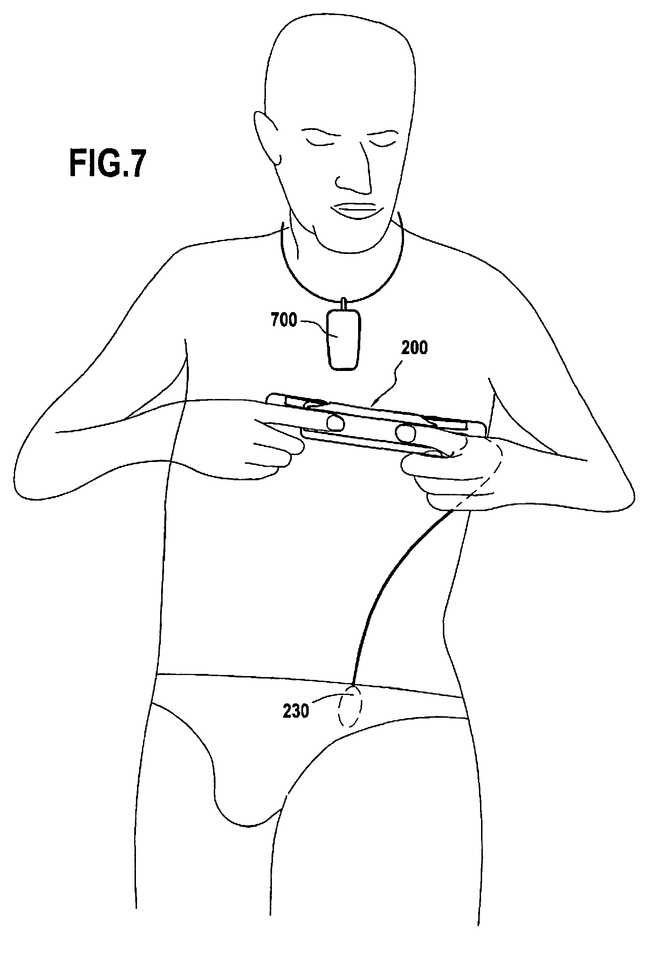

La **figure 7** illustre une vue schématique de l'utilisation de l'électrocardiographe 200. Le patient applique l'électrocardiographe 200 contre son thorax, en le tenant à deux mains. L'électrode périphérique 230 est disposée à l'aine gauche du patient. L'électrode neutre 207 est appliquée sous son mamelon gauche.

En variante, l'électrocardiographe 200 peut disposer d'un repère visuel ou en relief sur sa face supérieure 215 permettant au patient de positionner l'électrode neutre 207 au bon endroit (ex. en appliquant ce repère visuel sous le mamelon gauche).

Au cours de l'application de l'électrocardiographe 200 sur le thorax du patient, les bras 212 et 213 sont écartés de leurs positions de repos afin de former un angle d'ouverture avec la face latérale 211 adapté à la morphologie du patient. Les angles d'ouverture entre le bras 212 et la face latérale 211 (angle φ) et le bras 213 et la face latérale 211 (angle ψ) sont tels que les électrodes 201 à 206 sont positionnées sur les points V1 à V6 du thorax du patient. Le patient applique ses deux index ou ses deux majeurs sur les électrodes périphériques 208 et 209 de façon à bien tenir l'appareil.

Afin de s'assurer du bon contact des électrodes précordiales 201-206 avec les points V1-V6, des tests sont effectués sur le positionnement pour chaque électrode. En cas de mauvais positionnement de l'une des électrodes un message est affiché sur l'écran graphique 250.

Une fois que chaque électrode a été correctement positionnée, l'électrocardiographe 200 acquiert les signaux associés à chaque électrode et délivrer les douze dérivations de l'électrocardiogramme.

Par ailleurs dans un autre mode de réalisation, on acquiert à l'aide des électrodes précordiales de l'électrocardiographe selon l'invention des signaux relatifs à des points du thorax du patient différents des points V1 à V6. Un traitement numérique approprié est alors mis en oeuvre pour délivrer un électrocardiogramme à douze dérivations permettant à un médecin de diagnostiquer des anomalies.

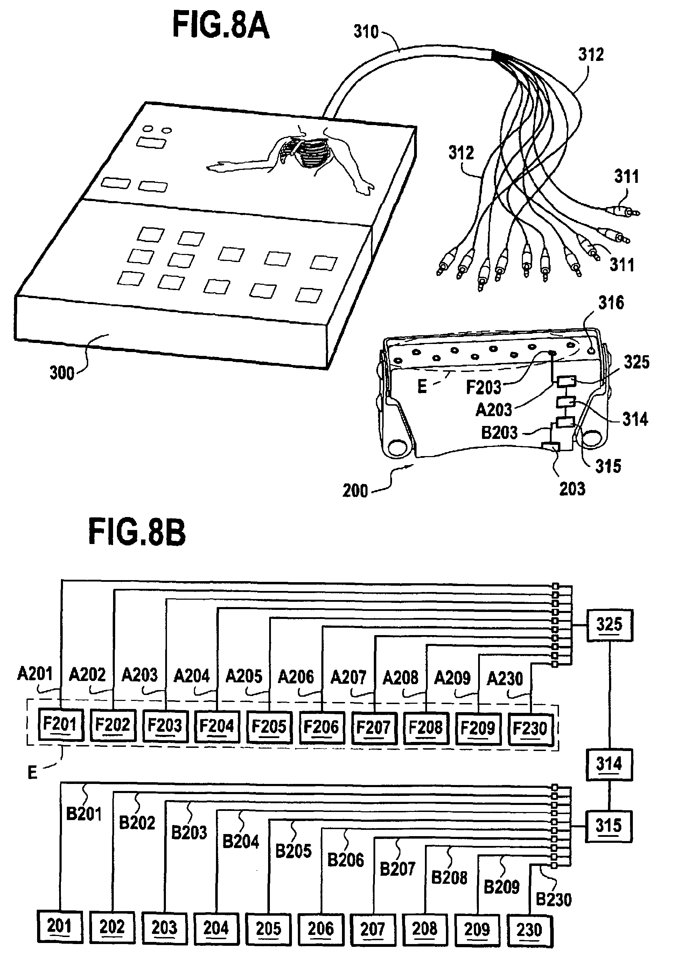

La **figure 8A** représente un électrocardiographe 200 conforme à un mode particulier de réalisation.

Celui-ci comporte, pour chacune de ses électrodes, des moyens pour rediriger le signal analogique obtenu par cette électrode vers un convertisseur analogique/numérique 315. Les signaux numérisés sont ensuite mémorisés dans une mémoire non volatile réinscriptible 314.

Lorsque l'utilisateur appuie sur un bouton 316, le signal numérisé correspondant à chacune des électrodes est lu de la mémoire 314 et converti en signal analogique par un convertisseur numérique/analogique 325.

Ce signal analogique est redirigé vers une fiche compatible avec un premier connecteur 311 d'un brin 312 dont un deuxième connecteur non représenté ici est compatible avec un autre électrocardiographe 300.

Dans le mode de réalisation décrit ici, tous ces brins 312 sont regroupés dans un même câble 310.

Dans le mode de réalisation décrit ici, l'électrocardiographe 200 comporte dix électrodes similaires aux électrodes 201 à 209 et 230 décrites précédemment. Pour des raisons de clarté, seule l'électrode 203 est représentée sur la figure 8A.

L'électrocardiographe 200 comporte ainsi un ensemble E de dix fiches chacune étant reliée à une des électrodes 201 à 209 et 230.

Pour des raisons de clarté, on a représenté sur cette figure :
- l'électrode 203 ;
- la liaison électrique B203 entre cette électrode et le convertisseur analogique/numérique 315 ;
- la mémoire non volatile réinscriptible 314 ;
- le convertisseur numérique/analogique 325 ; et
- la liaison électrique A203 entre ce convertisseur 325 et la fiche F203 correspondant à cette électrode 203.

Ainsi, les dix signaux analogiques obtenus par les électrodes 201 à 209 et 230 peuvent être fournis en entrée de l'électrocardiographe 300, celui-ci pouvant restituer les douze dérivations de l'électrocardiographe correspondant à ces signaux.

Ainsi, les signaux analogiques peuvent être fournis en entrée de l'électrocardiographe 300, un certain temps après leur acquisition, tant que le contenu de la mémoire n'a pas été effacé ou écrasé par une nouvelle acquisition. Dans un mode de réalisation particulier, la mémoire 314 a la capacité suffisante pour mémoriser des signaux de plusieurs acquisitions.

A la **figure 8B****,** on a représenté en détails :
- les liaisons électriques B201-B209, B230 entre les électrodes 201-209, 230 et le convertisseur analogique/numérique 315 ;
- la liaison électrique entre le convertisseur analogique numérique 315 et la mémoire 314 ;
- la liaison électrique entre la mémoire 314 et le convertisseur numérique/analogique 325 ; et
- les liaisons électriques A201-A209, A230 entre le convertisseur numérique/analogique 325 et les fiches F201-F209, F230.

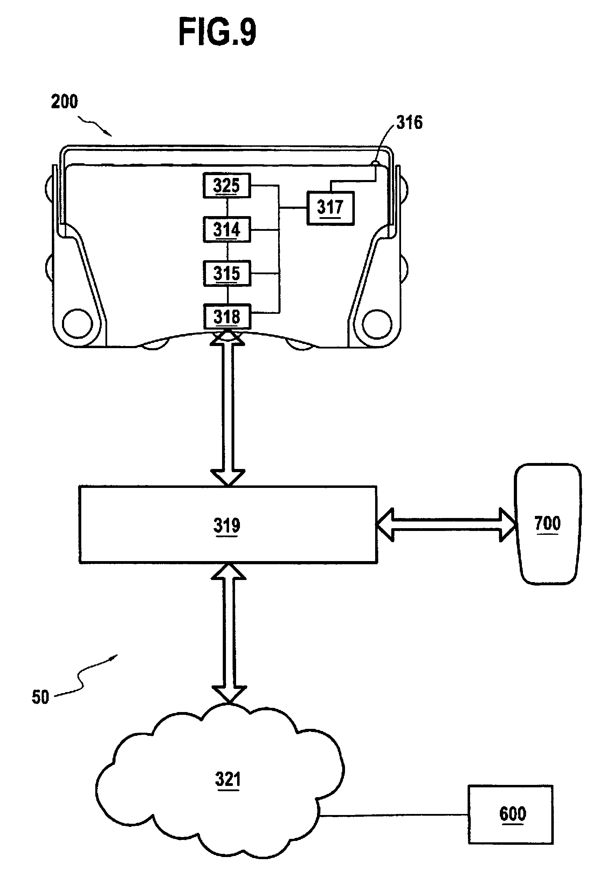

La **figure 9** représente un système de surveillance à distance de l'activité cardiaque d'un patient conforme à l'invention. Ce système 50 comporte principalement un électrocardiographe 200 conforme à un mode particulier de réalisation, une base Bluetooth (marque déposée) 319 connectée à un réseau de télécommunications 321, et un médaillon 700 apte à établir une communication Bluetooth, via la base 319, avec un opérateur se trouvant dans un centre distant 600.

Le patient peut par exemple porter le médaillon 700 autour de son cou comme représenté à la figure 7.

Dans le mode de réalisation décrit ici, l'électrocardiographe 200 a la structure générale d'un ordinateur. Il comporte notamment un processeur 317 et des moyens de télécommunication 318 aptes à établir une liaison Bluetooth (marque déposée) avec la base 319.

Dans le mode de réalisation décrit ici, l'électrocardiographe 200 comporte dix électrodes similaires aux électrodes 201 à 209 et 230 décrites précédemment, chacune de ces électrodes étant connectée à une mémoire non volatile réinscriptible 314 au moyen d'une liaison électrique B201-B209, B230 comme représenté à la figure 8B.

Dans le mode de réalisation décrit ici, pour éviter d'enregistrer des signaux non stabilisés, le processeur 317 est apte à déterminer si les signaux acquis par les électrodes sont conformes, et si c'est le cas à mémoriser ces signaux dans la mémoire 314 après conversion par un convertisseur analogique numérique 315 à celui déjà décrit.

A cet effet, le processeur 317 détermine si les signaux analogiques acquis par les électrodes ont des caractéristiques d'un complexe QRS.

Dans le mode de réalisation décrit ici, le processeur 317 est apte à filtrer les signaux déterminés comme étant conformes en mettant en oeuvre l'algorithme de Pan Tompkins. Il mémorise aussi ces signaux filtrés dans la mémoire 314.

Dans le mode de réalisation décrit ici, l'électrocardiographe ambulatoire événementiel 200 affiche sur l'écran 250 représenté à la figure 3, un message représentatif du bon et/ou du mauvais positionnement des électrodes.

Dans le mode de réalisation décrit ici, le processeur 317 est déclenche la transmission des signaux mémorisés dans la mémoire 314 vers le centre distant 600 dès que les signaux sont filtrés.

En variante, il pourrait transmettre les signaux détectés comme étant conformes, avant filtrage.

Pour cela il établit une liaison Bluetooth (marque déposée) entre ses moyens de communication 318 et la base 319, celle-ci relayant les signaux vers le centre distant 600.

Dans un autre mode de réalisation, l'électrocardiographe 200 peut être configuré pour ne pas transmettre automatiquement les signaux au centre distant 600, le processeur 317 vérifiant systématiquement cette configuration avant de déclencher un transfert.

Dans le mode de réalisation décrit ici, les signaux sont conservés dans la mémoire 314 tant que celle-ci n'est pas saturée.

Dans le mode de réalisation décrit ici, le processeur 317 est apte à détecter la réception d'une commande reçue, via la base Bluetooth 319, en provenance du centre distant 600, et à transmettre au centre distant 600, en réponse à cette commande, des signaux mémorisés dans la mémoire 314. Cette commande peut spécifier si les signaux à transmettre sont les signaux conformes avant ou après filtrage.

Dans le mode de réalisation décrit ici, l'électrocardiographe 200 comporte, comme décrit en référence à l'électrocardiographe des figures 8A et 8B, un ensemble E de dix fiches F201-F209, F230 permettant de fournir en sortie les signaux obtenus par les électrodes 201 à 209 et 230, vers un autre électrocardiographe 300.

Dans le mode de réalisation décrit ici, les dix fiches F201-F209, F230 sont également des fiches d'entrée constituant des moyens dits « secondaires » d'obtention d'une pluralité de signaux représentatifs de l'activité cardiaque d'un patient, ces signaux ayant été acquis par un autre dispositif, par exemple une ceinture d'électrodes.

A cet effet, on utilise des brins 312 pour véhiculer les signaux acquis par cet autre dispositif vers chacune les fiches F201-F209, F230 de l'électrocardiographe 200, ces brins étant terminés par un connecteur 311 compatible avec ces fiches.

Les signaux obtenus par ces moyens secondaires peuvent être mémorisés dans la mémoire 314 ou télétransmis au centre distant 600 via la base 319.

## Revendications

1. Electrocardiographe ambulatoire événementiel (200) adapté à saisir un électrocardiogramme à douze dérivations à partir de signaux électriques analogiques représentatifs de l'activité cardiaque d'un patient acquis à l'aide d'au moins neuf électrodes, ledit électrocardiographe (200) comportant une face frontale formant corps dudit électrocardiographe munie de deux électrodes précordiales et deux bras de part et d'autre dudit corps, chacun muni de deux électrodes précordiales, chaque bras formant un angle avec la face frontale de sorte que la face frontale et les deux bras définissent une surface sensiblement concave,
ledit électrocardiographe (200) étant **caractérisé en ce que** :
- chaque bras (212,213) peut être replié le long dudit corps (210) et est rotatif autour d'un axe sensiblement parallèle (Δ,Δ') à ladite face frontale (211) ;
ledit bras étant adapté à former un angle d'ouverture variable (φ,ψ) avec ladite face frontale (211) de sorte que ladite surface sensiblement concave (214) peut s'adapter à la morphologie du patient afin de permettre le bon positionnement desdites électrodes précordiales (201-206) sur le thorax dudit patient ;
et **en ce que** ledit électrocardiographe (200) comporte des moyens (242,243) permettant de maintenir ledit bon positionnement desdites électrodes précordiales sur le thorax dudit patient, ces moyens comportant un ressort (242, 243) associé à chaque bras (212, 213) dudit /4 électrocardiographe, chacun desdits ressorts étant fixé par une extrémité au corps de l'électrocardiographe et par l'autre extrémité au bras auquel il est associé.

2. Electrocardiographe ambulatoire événementiel (200) selon la revendication 1, **caractérisé en ce qu'**il comporte deux bras (212,213) de part et d'autre dudit corps (210), chaque bras étant muni de deux électrodes précordiales.

3. Electrocardiographe ambulatoire événementiel (200) selon la revendication 2, **caractérisé en ce que** :
- les deux électrodes précordiales (201,202) de l'un desdits bras (213) correspondent respectivement aux points V1 et V2 du thorax du patient ;
- les deux électrodes précordiales (205,206) de l'autre desdits bras (212) correspondent respectivement aux points V5 et V6 du thorax du patient ; et
- la face frontale (211) du corps (210) dudit électrocardiographe est munie de deux électrodes précordiales (203,204) correspondant respectivement aux points V3 et V4 du thorax du patient.

4. Electrocardiographe ambulatoire événementiel (200) selon l'une quelconque des revendications 1 à 3, **caractérisé en ce qu'**il comporte en outre parmi lesdites neuf électrodes, deux électrodes (208,209) permettant l'acquisition de signaux relatifs aux membres supérieurs dudit patient.

5. Electrocardiographe ambulatoire événementiel (200) selon la revendication 4, **caractérisé en ce que** lesdites deux électrodes (208,209) permettant l'acquisition des signaux relatifs aux membres supérieurs du patient sont disposées sur ledit électrocardiographe de façon à permettre l'application des index ou des majeurs dudit patient sur lesdites deux électrodes lorsque lesdites six électrodes précordiales sont positionnées sur le thorax dudit patient.

6. Electrocardiographe ambulatoire événementiel (200) selon l'une quelconque des revendications 1 à 5, **caractérisé en ce qu'**il comporte en outre parmi lesdites neuf électrodes une électrode (230) de type cuillère ou palette se trouvant à l'extrémité d'un cordon attaché audit électrocardiographe et permettant l'acquisition du signal relatif au membre inférieur gauche dudit patient.

7. Electrocardiographe ambulatoire événementiel (200) selon l'une quelconque des revendications 1 à 6, **caractérisé en ce qu'**il comporte en outre en sus desdites neuf électrodes une électrode neutre (207) positionnée sur ladite face frontale (211) dudit corps (210) dudit électrocardiographe de sorte à être appliquée sous le mamelon gauche dudit patient.

8. Electrocardiographe ambulatoire événementiel (200) selon l'une quelconque des revendications 1 à 7 , **caractérisé en ce qu'**il comporte, pour chacune desdites électrodes :
- des moyens (315) pour convertir le signal analogique acquis par cette électrode en signal numérique ;
- des moyens (314) pour mémoriser ledit signal numérique ;
- des moyens (325) pour convertir ledit signal numérique en signal analogique et (A201-A209, A230) pour rediriger ce signal analogique vers une fiche compatible avec un premier connecteur (311) d'un brin (312) dont un deuxième connecteur est compatible avec un deuxième électrocardiographe (300).

9. Electrocardiographe événementiel ambulatoire (200) selon l'une quelconque des revendications 1 à 8 , **caractérisé en ce qu'**il comporte :
- des moyens (317) aptes à déterminer si lesdits signaux analogiques comportent des caractéristiques correspondant à au moins un complexe QRS, de tels signaux étant dits "conformes" ; et
- une mémoire (314) apte à mémoriser lesdits signaux conformes.

10. Electrocardiographe événementiel ambulatoire (200) selon la revendication 9 , **caractérisé en ce qu'**il comporte des moyens de filtrage desdits signaux conformes.

11. Electrocardiographe événementiel ambulatoire (200) selon la revendication 9 ou 10, **caractérisé en ce qu'**il comporte :
- des moyens (318) de télécommunication aptes à transmettre lesdits signaux conformes ou filtrés à un centre distant (600) à partir de ladite mémoire (314) ; et
- des moyens de contrôle (317) aptes à déclencher ladite transmission sur détection d'au moins un événement prédéterminé.

12. Electrocardiographe selon la revendication 11, **caractérisé en ce que** lesdits moyens de contrôle (317) sont adaptés à déclencher ladite transmission dès que lesdits signaux ont été déterminés comme étant conformes ou dès qu'ils ont été filtrés.

13. Electrocardiographe selon la revendication 11 ou 12, **caractérisé en ce que** lesdits moyens de contrôle (317) sont adaptés à déclencher ladite transmission suite à la réception, par ledit électrocardiographe (200), d'une commande à cet effet.

14. Electrocardiographe selon l'une quelconque des revendications 11 à 13, **caractérisé en ce que** lesdits moyens de contrôle sont aptes à empêcher ladite transmission.

15. Electrocardiographe selon l'une quelconque des revendications 1 à 14 , **caractérisé en ce qu'**il comporte des moyens secondaires d'obtention d'une pluralité de signaux représentatifs de l'activité cardiaque d'un patient en provenance d'un autre dispositif.

16. Electrocardiographe selon la revendication 15, **caractérisé en ce que** lesdits moyens secondaires comportent une pluralité de fiches (F201-F209, F230), chacune étant compatible avec un connecteur (311) d'un brin (312) apte à véhiculer un signal analogique obtenu par ledit dispositif.

17. Electrocardiographe ambulatoire événementiel (200) selon l'une quelconque des revendications 1 à 16, comportant en outre des moyens (250) pour afficher un message représentatif du bon et/ou du mauvais positionnement des électrodes.

18. Système (50) de surveillance à distance de l'activité cardiaque d'un patient comportant :
- un électrocardiographe (200) selon l'une quelconque des revendications 11 à 14, dans lequel lesdits moyens de télécommunication (318) sont constitués par des moyens de télécommunication sans fil avec une base (319) connectée à un réseau de télécommunication ; et
- ladite base (319).

19. Système de surveillance selon la revendication 18, **caractérisé en ce qu'**il comporte en outre un équipement portatif (700) apte à établir une communication vocale, via ladite base (319), avec un opérateur distant.

## Claims

1. An event-driven ambulatory electrocardiograph (200) adapted for acquiring a twelve-derivative electrocardiogram from electric analog signals representative of the cardiac activity of a patient acquired using at least nine electrodes, said electrocardiograph (200) comprising a front face forming the body of said electrocardiograph fitted with two precordial electrodes and two arms on either side of said body, each fitted with two precordial electrodes, each arm forming an angle with the front face such that the front face and the two arms define a substantially concave surface,
said electrocardiograph (200) being **characterised in that**:
- each arm (212, 213) can be folded back along said body (210) and can rotate around an axis substantially parallel (Δ, Δ') to said front face (211);
said arm being adapted to form an angle of variable opening (φ, ψ) with said front face (211) such that said substantially concave surface (214) can adapt to the morphology of the patient to allow proper positioning of said precordial electrodes (201-206) on the thorax of said patient;
and **in that** said electrocardiograph (200) comprises means (242, 243) for maintaining said proper positioning of said precordial electrodes on the thorax of said patient, these means comprising a spring (242, 243) associated with each arm (212, 213) of said electrocardiograph, each of said springs being fixed by one end to the body of the electrocardiograph and by the other end to the arm with which it is associated.

2. The event-driven ambulatory electrocardiograph (200) according to Claim 1, **characterised in that** it comprises two arms (212, 213) on either side of said body (210), each arm being fitted with two precordial electrodes.

3. The event-driven ambulatory electrocardiograph (200) according to Claim 2, **characterised in that**:
- the two precordial electrodes (201, 202) of one of said arms (213) correspond respectively to points V1 and V2 of the thorax of the patient;
- the two precordial electrodes (205, 206) of the other of said arms (212) correspond respectively to points V5 and V6 of the thorax of the patient; and
- the front face (211) of the body (210) of said electrocardiograph is fitted with two precordial electrodes (203, 204) corresponding respectively to points V3 and V4 of the thorax of the patient.

4. The event-driven ambulatory electrocardiograph (200) according to any one of Claims 1 to 3, **characterised in that** it further comprises of said nine electrodes two electrodes (208, 209) permitting acquisition of signals relative to the upper members of said patient.

5. The event-driven ambulatory electrocardiograph (200) according to Claim 4, **characterised in that** said two electrodes (208, 209) for acquiring signals relative to the upper members of the patient are arranged on said electrocardiograph to allow application of the index or middle fingers of said patient on said two electrodes when said six precordial electrodes are positioned on the thorax of said patient.

6. The event-driven ambulatory electrocardiograph (200) according to any one of Claims 1 to 5, **characterised in that** it further comprises of said nine electrodes, one electrode (230) of spoon or palette type located at the end of a cordon attached to said electrocardiograph and allowing acquisition of the signal relative to the lower left member of said patient.

7. The event-driven ambulatory electrocardiograph (200) according to any one of Claims 1 to 6, **characterised in that** it further comprises above said nine electrodes, a neutral electrode (207) positioned on said front face (211) of said body (210) of said electrocardiograph so as to be applied under the left nipple of said patient.

8. The event-driven ambulatory electrocardiograph (200) according to any one of Claims 1 to 7, **characterised in that** it comprises, for each of said electrodes:
- means (315) for converting the analog signal acquired by this electrode to a digital signal;
- means (314) for storing said digital signal;
- means (325) for converting said digital signal into an analog signal and (A201-A209, A230) for redirecting this analog signal to a chip compatible with a first connector (311) of a strand (312) whereof a second connector is compatible with a second electrocardiograph (300).

9. The event-driven ambulatory electrocardiograph (200) according to any one of Claims 1 to 8, **characterised in that** it comprises:
- means (317) capable of determining whether said analog signals comprise characteristics corresponding to at least one QRS complex, such signals being known as "compatible"; and
- a memory (314) capable of storing said compatible signals.

10. The event-driven ambulatory electrocardiograph (200) according to Claim 9, **characterised in that** it comprises filtering means of said compatible signals.

11. The event-driven ambulatory electrocardiograph (200) according to Claim 9 or 10, **characterised in that** it comprises:
- telecommunications means (318) capable of transmitting said compatible signals or filtered to a remote centre (600) from said memory (314); and
- control means (317) capable of triggering said transmission on detection of at least one predetermined event.

12. The electrocardiograph according to Claim 11, **characterised in that** said control means (317) are adapted to trigger said transmission as soon as said signals have been determined as being compatible or as soon as they have been filtered.

13. The electrocardiograph according to Claim 11 or 12, **characterised in that** said control means (317) are adapted to trigger said transmission following receipt reception, by said electrocardiograph (200), of a command for this purpose.

14. The electrocardiograph according to any one of Claims 11 to 13, **characterised in that** said control means are capable of preventing said transmission.

15. The electrocardiograph according to any one of Claims 1 to 14, **characterised in that** it comprises secondary means for obtaining a plurality of signals representative of the cardiac activity of a patient originating from another device.

16. The electrocardiograph according to Claim 15, **characterised in that** said secondary means comprise a plurality of chips (F201-F209, F230), each being compatible with a connector (311) of a strand (312) for conveying an analog signal obtained by said device.

17. The event-driven ambulatory electrocardiograph (200) according to any one of Claims 1 to 16, further comprising means (250) for displaying a message representative of the correct and/or incorrect positioning of the electrodes.

18. A remote monitoring system (50) of the cardiac activity of a patient comprising:
- an electrocardiograph (200) according to any one of Claims 11 to 14, in which said telecommunications means (318) comprise wireless telecommunications means with a base (319) connected to a telecommunications network; and
- said base (319).

19. The monitoring system according to Claim 18, **characterised in that** it further comprises portable equipment (700) capable of setting up voice communication, via said base (319), with a remote operator.

## Patentansprüche

1. Ambulanter Ereignis-Elektrokardiograph (200), der dafür ausgelegt ist, ein 12-Kanal-EKG anhand von für die Herztätigkeit eines Patienten repräsentativen elektrischen Analogsignalen, die mit Hilfe von wenigstens neun Elektroden aufgenommen werden, zu erfassen, wobei der Elektrokardiograph (200) eine einen Bestandteil des Elektrokardiographen bildende Stirnfläche, die mit zwei präkordialen Elektroden ausgestattet ist, sowie zwei Arme auf beiden Seiten des Körpers umfaßt, die jeweils mit zwei präkordialen Elektroden ausgestattet sind, wobei jeder Arm mit der Stirnfläche einen Winkel bildet, so daß die Stirnfläche und die beiden Arme eine im wesentlichen konkave Fläche bilden,
wobei der Elektrokardiograph (200) **dadurch gekennzeichnet ist, daß**:
- jeder Arm (212, 213) entlang dem Körper (210) eingeklappt werden kann und um eine zu der Stirnfläche (211) im wesentlichen parallele Achse (Δ, Δ') drehbar ist,
wobei der Arm dafür ausgelegt ist, einen veränderlichen Öffnungswinkel (φ, ψ) mit der Stirnfläche (211) zu bilden, so daß die im wesentlichen konkave Fläche (214) sich der Morphologie des Patienten anpassen kann, um die richtige Positionierung der präkordialen Elektroden (201-206) am Brustkorb des Patienten zu ermöglichen,
und daß der Elektrokardiograph (200) Mittel (242, 243) umfaßt, die ermöglichen, die richtige Positionierung der präkordialen Elektroden am Brustkorb des Patienten beizubehalten, wobei diese Mittel eine jedem Arm (212, 213) des Elektrokardiographen zugeordnete Feder (242, 243) umfassen, wobei eine jede der Federn mit einem Ende an dem Körper des Elektrokardiographen und mit dem anderen Ende an dem Arm, dem sie zugeordnet ist, befestigt ist.

2. Ambulanter Ereignis-Elektrokardiograph (200) nach Anspruch 1, **dadurch gekennzeichnet, daß** er zwei Arme (212, 213) auf beiden Seiten des Körpers (210) umfaßt, wobei jeder Arm mit zwei präkordialen Elektroden ausgestattet ist.

3. Ambulanter Ereignis-Elektrokardiograph (200) nach Anspruch 2, **dadurch gekennzeichnet, daß**:
- die beiden präkordialen Elektroden (201, 202) von einem der Arme (213) den Steifen V1 bzw. V2 des Brustkorbs des Patienten entsprechen,
- die beiden präkordialen Elektroden (205, 206) des anderen der Arme (212) den Stellen V5 bzw. V6 des Brustkorbs des Patienten entsprechen und
- die Stirnfläche (211) des Körpers (210) des Elektrokardiographen mit zwei präkordialen Elektroden (203, 204) ausgestattet ist, die den Stellen V3 bzw. V4 des Brustkorbs des Patienten entsprechen.

4. Ambulanter Ereignis-Elektrokardiograph (200) nach einem der Ansprüche 1 bis 3, **dadurch gekennzeichnet, daß** er ferner unter den neun Elektroden zwei Elektroden (208, 209) umfaßt, die die Aufnahme von Signalen bezüglich der oberen Gliedmaßen des Patienten ermöglichen.

5. Ambulanter Ereignis-Elektrokardiograph (200) nach Anspruch 4, **dadurch gekennzeichnet, daß** die beiden Elektroden (208, 209), die die Aufnahme der Signale bezüglich der oberen Gliedmaßen des Patienten ermöglichen, an dem Elektrokardiographen derart angeordnet sind, daß sie das Anlegen der Zeigefinger oder der Mittelfinger des Patienten an die beiden Elektroden ermöglichen, wenn die sechs präkordialen Elektroden auf dem Brustkorb des Patienten positioniert sind.

6. Ambulanter Ereignis-Elektrokardiograph (200) nach einem der Ansprüche 1 bis 5, **dadurch gekennzeichnet, daß** er ferner unter den neun Elektroden eine löffel- oder schaufelartige Elektrode (230) umfaßt, die sich am Ende einer an dem Elektrokardiographen befestigten Schnur befindet und die Aufnahme des Signals bezüglich der linken unteren Gliedmaße des Patienten ermöglicht.

7. Ambulanter Ereignis-Elektrokardiograph (200) nach einem der Ansprüche 1 bis 6, **dadurch gekennzeichnet, daß** er ferner zusätzlich zu den neun Elektroden eine neutrale Elektrode (207) umfaßt, die an der Stirnfläche (211) des Körpers (210) des Elektrokardiographen derart positioniert ist, daß sie unter der linken Brustwarze des Patienten angelegt wird.

8. Ambulanter Ereignis-Elektrokardiograph (200) nach einem der Ansprüche 1 bis 7, **dadurch gekennzeichnet, daß** er für jede der Elektroden umfaßt:
- Mittel (315) zum Umwandeln des durch diese Elektrode aufgenommenen analogen Signals in ein digitales Signal,
- Mittel (314) zum Speichern des digitalen Signals,
- Mittel (325) zum Umwandeln des digitalen Signals in ein analoges Signal sowie (A201-A209, A230) zum Zurückleiten dieses analogen Signals zu einem Steckanschluß, der mit einem ersten Verbinder (311) einer Ader (312) kompatibel ist, von der ein zweiter Verbinder mit einem zweiten Elektrokardiographen (300) kompatibel ist.

9. Ambulanter Ereignis-Elektrokardiograph (200) nach einem der Ansprüche 1 bis 8, **dadurch gekennzeichnet, daß** er umfaßt:
- Mittel (317), die geeignet sind, zu bestimmen, ob die analogen Signale Merkmale aufweisen, die wenigstens einem QRS-Komptex entsprechen, wobei derartige Signale sogenannte "konforme Signale" sind, und
- einen Speicher (314), der geeignet ist, die konformen Signale zu speichern.

10. Ambulanter Ereignis-Elektrokardiograph (200) nach Anspruch 9, **dadurch gekennzeichnet, daß** er Mittel zur Filterung der konformen Signale umfaßt.

11. Ambulanter Ereignis-Elektrokardiograph (200) nach Anspruch 9 oder 10, **dadurch gekennzeichnet, daß** er umfaßt:
- Telekommunikationsmittel (318), die geeignet sind, die konformen oder gefilterten Signale von dem Speicher (314) aus an eine entfernte Zentrale (600) zu übertragen, sowie
- Steuermittel (317), die geeignet sind, die Übertragung bei Erfassen wenigstens eines vorbestimmten Ereignisses auszulösen.

12. Elektrokardiograph nach Anspruch 11, **dadurch gekennzeichnet, daß** die Steuermittel (317) dafür ausgelegt sind, die Übertragung auszulösen, sobald die Signale als konform bestimmt worden sind oder sobald sie gefiltert worden sind.

13. Elektrokardiograph nach Anspruch 11 oder 12, **dadurch gekennzeichnet, daß** die Steuermittel (317) dafür ausgelegt sind, die Übertragung infolge des Empfangs - durch den Elektrokardiographen (200)- eines Befehls hierzu auszulösen.

14. Elektrokardiograph nach einem der Ansprüche 11 bis 13, **dadurch gekennzeichnet, daß** die Steuermittel geeignet sind, die Übertragung zu verhindern.

15. Elektrokardiograph nach einem der Ansprüche 1 bis 14, **dadurch gekennzeichnet, daß** er sekundäre Mittel zum Erhalten einer Vielzahl von für die Herztätigkeit eines Patienten repräsentativen Signalen von einer anderen Vorrichtung umfaßt.

16. Elektrokardiograph nach Anspruch 15, **dadurch gekennzeichnet, daß** die sekundären Mittel eine Vielzahl von Steckanschlüssen (F201-F209, F230) umfassen, wobei ein jeder mit einem Verbinder (311) einer Ader (312), die geeignet ist, ein durch die Vorrichtung erhaltenes analoges Signal zu leiten, kompatibel ist.

17. Ambulanter Ereignis-Elektrokardiograph (200) nach einem der Ansprüche 1 bis 16, der ferner Mittel (250) zum Anzeigen einer für das richtige und/oder das falsche Positionieren der Elektroden repräsentativen Meldung umfaßt.

18. System (50) zur Fernüberwachung der Herztätigkeit eines Patienten, umfassend:
- einen Elektrokardiographen (200) nach einem der Ansprüche 11 bis 14, wobei die Telekommunikationsmittel (318) durch drahtlose Telekommunikationsmittel mit einer Basis (319), die an ein Telekommunikationsnetz angeschlossen ist, gebildet sind, sowie
- die Basis (319).

19. Überwachungssystem nach Anspruch 18, **dadurch gekennzeichnet, daß** es ferner eine tragbare Einrichtung (700) umfaßt, die geeignet ist, eine Sprachverbindung über die Basis (319) mit einer entfernten Bedienungsperson herzustellen.
